# EUROPEAN PATENT APPLICATION

(11) **EP 4 331 351 A1**
(43) Date of publication of application: **06.03.2024**
(21) Application number: 22795066.4
(22) Date of filing: 21.04.2022
(51) Int. Cl.: A01H 3/04

(54) **COMPOSITION FOR REDUCING ABIOTIC STRESS IN PLANTS AND IMPROVING PLANT METABOLISM EFFICIENCY**

(30) Priority: 27.04.2021 ES 202130369
(71) Applicant: Gestión Ecosistemas Agrícolas, S.L, 46018 Valencia (ES)
(72) Inventor: MARCO CASANOVA, Aurelio José, 46018 Valencia (ES)
(74) Representative: Ungria López, Javier
(86) International application number: PCT/ES2022/070243
(87) International publication number: WO 2022/229482

(57) **Abstract**

In a first aspect, the present invention relates to a composition for reducing abiotic stress in plants, characterised in that it comprises cis-3-hexenyl propionate as an active ingredient. A second aspect of the present invention relates to the method for producing the composition for reducing abiotic stress in plants. The present invention further relates to the dilution comprising the composition of the invention in an aqueous medium. Another aspect of the invention relates to the method for reducing abiotic stress, comprising applying the composition of the invention or the dilution at the foliar level and/or at the root level of the plants. A final aspect of the invention is the use of the composition or dilution described in the present document for reducing abiotic stress in plants and increasing metabolism.

## Description

### SECTOR OF THE ART

The present invention falls within the chemical and agricultural sector. More particularly, it relates to a new chemical composition for regulating abiotic stress in plants.

### BACKGROUND OF THE INVENTION

There are different types of environmental stress (abiotic), such as drought, salinity, high degrees of light and extreme temperatures that influence the growth and productivity of plants. These environmental factors damage vital processes, such as photosynthesis and protein synthesis, which results in a decrease in plant growth and biomass production.

Alterations also occur in the hormonal balance and oxidation/reduction processes, which damage DNA and, as a result, the proteins and lipids of cell membranes.

Therefore, throughout evolution, plants have developed mechanisms to adapt to these abiotic stresses. These mechanisms repress growth mechanisms and trigger protection and defence mechanisms that compromise their development, but they ensure the survival of the plant under adverse environmental conditions. Many of these mechanisms are related to a greater capacity to absorb water or a more efficient use of resources.

Plant hormones are responsible for coordinating these processes of growth and differentiation of plant tissues, in addition to adjusting the response of the plants to different types of abiotic stress. Among the most important hormones, abscisic acid (ABA) is the key hormone in the response of plants to water stress, caused by both lack of water and high salinity. In these conditions, endogenous ABA content increases, causing a series of metabolic and biochemical responses in the plant, limiting their growth and decreasing their metabolic activity, as well as their transpiration. In general, salinity limits plant growth by decreasing transpiration and photosynthetic capacity of plants.

Thus, the agricultural sector considers it necessary to develop different products and mechanisms that can be applied to plants in order to avoid, as much as possible, the response mechanisms of plants to abiotic stress and thereby ensure that plants continue to experience good growth rates, despite environmental conditions.

In the state of the art, different inventions have been found related to improving the tolerance of plants to abiotic stress.

In this sense, British application GB2090585A relates to a composition to give physico-chemical and biological stability to plants, comprising tetravalent titanium ions along with sorbic acid and salts thereof, benzoic acid and salts thereof, salicylic acid and salts thereof, propionic acid and salts thereof, p-nitrobenzoic acid and salts thereof and hexamethylenetetramine.

Another example is the international application WO2017216003A1 which relates to the improvement of abiotic stress tolerance in rice plants by administering a composition, and in particular, to the stress caused in plants by high temperatures. This patent application describes the application of a chemical composition, but the results thereof are limited to rice plants.

Moreover, patent application US2015247161A1 relates to a method for producing abiotic stress resistant plants that over-express OsAlba1. Although this application solves the problem of making plants more resistant to water stress, the way it does so is by producing transgenic plants.

Based on the foregoing, it can be stated that up to the present, an innocuous composition that improves the adaptation of plants to stress, effectively improving the metabolism of plants, with a surprisingly high plant growth rate, as described in this patent document, has not been known.

The composition object of the invention solves this need, having unique properties, compared to other chemical compositions known in the state of the art for controlling abiotic stress in the metabolism of plants.

### BRIEF DESCRIPTION OF THE INVENTION

Thus, a first aspect of the present invention is a composition for treating abiotic stress in plants, characterised in that it comprises, in percentage by weight with respect to the total composition:
cis-3-hexenyl propionate comprised between 3% and 50%;
at least one glycol comprised between 49.5% and 85%;
at least one emulsifying product comprised between 0% and 10%;
at least one dispersant product comprised between 0% and 2%,
at least one preservative additive comprised between 0% and 2%,
the sum of the components being less than or equal to 100%.

In the context of the present invention, abiotic stress is understood as a series of environmental conditions, such as drought, salinity, high degrees of light and extreme temperatures that influence the growth and productivity of plants. Therefore, abiotic stress treatment is understood as all types of actions that one can do to a plant to improve the adaptation mechanisms of plants in situations of abiotic stress, keeping the plant's metabolism active and, as a final result, maintaining and even improving the growth rate thereof, with respect to standards in favourable environmental conditions.

Likewise, in the context of the present invention, the regulation of plants' metabolisms is understood as all types of human actions on the different metabolic pathways that intervene in the growth and development of plants to maintain and even improve the growth rate thereof.

Furthermore, in the context of the present invention, the term plants refers to plants of herbaceous species, plants, vegetables or fruit trees in general, which are found in agricultural crops, without being limited exclusively to plants.

The composition object of the invention is characterised in that it has a viscosity between 1 to 100 mPas at a temperature of 25°C.

In a second aspect, the present invention relates to a method for producing the composition described herein, which is characterised in that it comprises mixing an amount comprised between 3% and 50% of cis-3-hexenyl propionate with an amount comprised between 49.5% and 85% of at least one water-soluble glycol while stirring at a speed of 100 to 300 rpm at a temperature between 20 to 30°C for a time of 20 to 30 minutes, the quantities being by weight with respect to the total of the composition object of the invention.

In a third aspect, the invention relates to a dilution comprising between 0.01% to 10% of the composition object of the invention in a liquid medium.

In another aspect of the present invention, the method for reducing abiotic stress is also described, which comprises applying the composition object of the invention or the dilution thereof to agricultural crops at the foliar level of the plants by means of spraying, fumigation or controlled evaporation.

The object of the invention is also the method for reducing abiotic stress comprising applying the composition object of the invention or the dilution thereof to agricultural crops to the root level of the plants through irrigation.

A final aspect of the present invention is the use of the composition described herein or the dilution thereof to reduce abiotic stress and increase the metabolism of plants.

### DETAILED DESCRIPTION OF THE INVENTION

Thus, a first aspect of the present invention is a composition for reducing abiotic stress in plants, characterised in that it comprises, in percentage by weight with respect to the total composition:
cis-3-hexenyl propionate comprised between 3% and 50%;
at least one glycol comprised between 49.5% and 85%;
at least one emulsifying product comprised between 0% and 10%;
at least one dispersant product comprised between 0% and 2%,
at least one preservative additive comprised between 0% and 2%,
the sum of the components being less than or equal to 100%.

In a particular embodiment, the composition object of the invention comprises cis-3-hexenyl propionate in an amount comprised between 3% - 50%, preferably between 4% - 30%, and even more preferably between 5% - 20%. In other particular embodiments, the composition object of the invention can comprise 10%, 20%, 30%, 40%, or 50% cis-3-hexenyl propionate by weight with respect to the total composition.

In another particular embodiment of the present invention, the composition object of the invention comprises at least one glycol in an amount comprised between 49.5% and 85%, preferably between 60% and 80%, and even more preferably between 65% and 75%. In other particular embodiments, the composition object of the invention can comprise 50 %, 60%, 70% or 80% by weight, with respect to the total composition, of at least one glycol. In another particular embodiment of the present invention, the glycol comprising the composition can be selected from the group of ethylene or propylene glycols. In particular embodiments of the present invention, the glycol is selected, although not limited to, the group consisting of monoethylene glycol, diethylene glycol, triethylene glycol, monopropylene glycol, dipropylene glycol, glycerin or a combination thereof. In even more preferred embodiments of the present invention, the glycol is selected from the group consisting of monoethylene glycol, diethylene glycol, triethylene glycol, monopropylene glycol, dipropylene glycol, glycerin and a combination thereof. In more preferred embodiments, the glycol is selected from the group consisting of monoethylene glycol, monopropylene glycol, diethylene glycol and a combination thereof.

In particular, the composition object of the invention can comprise at least one additive that is selected from the group consisting of emulsifiers, dispersants, preservatives and a combination thereof.

The composition object of the invention is characterised in that it has a viscosity between 1 to 100 mPas at a temperature of 25°C. In particular embodiments, the composition object of the invention has a viscosity comprised between 5 and 80 mPas, preferably between 35 and 75 mPas and even more preferably between 45 and 65 mPas. In other particular embodiments, the composition object of the invention is characterised in that it can have a viscosity of 10, 20, 30, 40, 50, 60, 70, 80 or 90 mPas at a temperature of 25°C.

In a particular embodiment of the present invention, at least one emulsifying additive is used, the function of which is to promote the formation of an emulsion during the dilution process prior to the application thereof. The emulsifying additives that can be used in the present invention can be of different nature. Some examples of additives that can be used, which are non-limiting with regard to the invention, are lecithin, polysorbate, ammonium phosphate, guar flour, glyceryl esters, fatty acid salts, fatty acid monoglycerides and diglycerides, citric acid, tartaric or acetic esters, polyglyceride esters, fatty acids and glutamates. In a particular embodiment of the present invention, the composition object of the invention can comprise at least one emulsifying additive that is selected from the group consisting of lecithin, polysorbate, ammonium phosphate, guar flour, glyceryl esters, fatty acid salts, fatty acid monoglycerides or diglycerides, citric acid, tartaric or acetic esters, polyglyceride esters, fatty acids, glutamates and a combination thereof. The composition object of the invention comprises at least one emulsifying additive in a concentration comprised between 0% and 10% by weight with respect to the total composition, preferably between 1% and 5%, and in an even more preferred embodiment between 2% and 4%. In particular embodiments, the composition object of the invention can comprise 0.1%, 0.2%, 0.3%, 0.4%, 0.5%, 0.6%, 0.7%, 0.8% or 0.9% by weight, with respect to the total composition, of at least one emulsifying additive. In other particular embodiments, the composition object of the invention can comprise 1 %, 2%, 3%, 4%, 5%, 6%, 7%, 8%, 9% or 10% by weight, with respect to the total composition, of at least one emulsifying additive.

In a particular embodiment of the present invention at least one dispersant additive is used to favour the dispersion of the emulsion phases in the dilution stage prior to the application. The dispersant additives that can be used in the present invention can be of different nature. Some examples of additives that can be used, which are non-limiting with regard to the invention are, as inorganic additives, calcium or barium sulphates, calcium carbonate, barium or magnesium, sodium phosphate, or calcium and, as organic additives, polyvinyl alcohol, methyl cellulose, methyl hydroxypropyl cellulose, ethyl cellulose and sodium carboxymethyl cellulose. In a particular embodiment of the present invention, the composition object of the invention can comprise at least one inorganic dispersant additive selected from the group consisting of calcium or barium sulphates, calcium carbonate, barium or magnesium, sodium or calcium phosphates and a combination thereof. In other particular embodiments of the present invention, the composition object of the invention can comprise at least one organic dispersant additive selected from the group consisting of polyvinyl alcohol, methyl cellulose, methyl hydroxypropyl cellulose, ethyl cellulose, sodium carboxymethyl cellulose and a combination thereof. In particular embodiments of the present invention, the composition object of the invention can comprise a combination of organic and inorganic additives. The composition object of the invention comprises the dispersant additive in a concentration comprised between 0% and 2% by weight with respect to the total composition, preferably between 0.05% and 1.5%, and in an even more preferred embodiment between 0.1% and 1% by weight with respect to the total composition. In other particular embodiments, the composition object of the invention can comprise 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9% or 2% by weight with respect to the total composition of at least one dispersant additive.

In a particular embodiment of the present invention, preservative additives can optionally be used to extend the durability of the composition during storage time. The preservative additives that can be used in the present invention can be of different nature. Some examples of additives that can be used, which are non-limiting with regard to the invention, are sorbic acid, sodium sorbate, benzoic acid, sodium benzoate, formic acid, sodium formate, boric acid, potassium tetraborate, acetic acid, sodium or potassium acetate, lactic acid, and chitosan. In particular embodiments of the present invention, the composition object of the invention can comprise at least one preservative additive selected from the group consisting of sorbic acid, sodium sorbate, benzoic acid, sodium benzoate, formic acid, sodium formate, boric acid, potassium tetraborate, acetic acid, sodium or potassium acetate, lactic acid, chitosan and a combination thereof. The composition object of the invention can comprise the preservative additive in a concentration comprised between 0% and 2% by weight with respect to the total composition, preferably between 0.05% and 1.5%, and in an even more preferred embodiment between 0.1% and 1%. In other particular embodiments, the composition object of the invention can comprise 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9% or 2% by weight with respect to the total composition of at least one preservative additive.

The composition object of the invention intervenes in the proline metabolic pathway, a known metabolite related to tolerance to water stress and a natural osmolyte responsible for mediating stress tolerance by helping in the osmotic adjustment of plants.

The composition object of the invention acts by modifying hormonal levels, adapting the plant response to abiotic stress and improving the plant's tolerance to this adverse condition.

In a second aspect, the present invention relates to a method for producing the composition described herein, which is characterised in that it comprises mixing an amount comprised between 3% and 50% of cis-3-hexenyl propionate with an amount comprised between 49.5% and 85% of at least one water-soluble glycol while stirring at a speed of 100 to 300 rpm at a temperature between 20 to 30°C for a time of 20 to 30 minutes, the quantities being by weight with respect to the total of the composition object of the invention.

In particular embodiments, the stirring speed is comprised between 150 to 250 rpm, more preferably between 175 and 200 rpm. In other particular embodiments, the stirring time is comprised between 20 and 28 minutes, and more preferably, between 22 and 25 minutes.

In particular embodiments of the present invention, the method for producing the composition can comprise an additional previous step wherein;
at least one emulsifying additive in an amount comprised between 0.1%, and 10%, and/or
at least one dispersant additive in an amount comprised between 0.05% and 2%, and/or
at least one preservative additive in a concentration between 0.05% and 2%, the amounts by weight being with respect to the total composition,
are added to an amount comprised between 49.5% and 85% of at least one water-soluble glycol and are mixed at a speed of 100 to 300 rpm for a time between 20 and 60 minutes at a temperature between 20 and 30°C.

In a particular embodiment of the method of the invention, once the glycol is mixed with at least one additive, which can be the emulsifying additive, the dispersant additive, the preservative additive and a combination thereof, in the conditions mentioned in the previous paragraph, it is then mixed with cis-3-hexenyl propionate, which is comprised in an amount between 3% and 50%, creating the composition object of the invention.

In a third aspect, the invention relates to a dilution comprising the composition object of the invention in an amount between 0.01% to 10% by weight with respect to the total dilution, in a liquid medium. In a particular embodiment of the present invention, the liquid medium used in the present invention is pure water. In another particular embodiment, the liquid medium used is water mixed with fertilisers and/or agricultural additives. In a preferred embodiment, the water is mixed with an amount comprised between 0.01% and 10% by weight with respect to the total liquid medium of fertilizers and/or agricultural additives.

In particular embodiments, the dilution can comprise the composition object of the invention in an amount of 0.1%, 0.2%, 0.3%, 0.4%, 0.5%, 0.6%, 0.7%, 0.8%, 0.9% or 1% by weight with respect to the total dilution. In other particular embodiments, the dilution can comprise the composition object of the invention 1%, 2%, 3%, 4%, 5%, 6%, 7%, 8% or 9% by weight with respect to the total.

In the technical field of the present invention, fertilisers are understood to be a set of products used in agriculture or gardening which, due to the nutrient content thereof, facilitate plant growth, increase the yield thereof and improve the quality of crops.

Likewise, in the technical field of the present invention, agricultural additives are understood as the products used to improve crops by avoiding the presence of pests in the plants or favouring the fixation of nutrients.

In another aspect, the invention relates to the method for reducing abiotic stress and the metabolism, comprising applying the composition or dilution object of the invention at the foliar level and is carried out by techniques that are known in the state of the art for both high and low volume, such as spraying, fumigation or controlled evaporation.

The object of the invention is also a method for reducing abiotic stress comprising applying the composition or the dilution thereof object of the invention at the root level of the plants using techniques known in the state of the art that use a liquid medium as a vehicle for transporting the treatment, such as irrigation.

In particular embodiments of the method for reducing abiotic stress and modulating the metabolism, the composition object of the invention is applied at the foliar level in an amount comprised between 0.001 and 1000 litres per hectare (L/Ha) of crops through controlled evaporation, which, in another particular embodiment, is an amount comprised between 0.1 and 1000 L/Ha. In preferred embodiments, the amount of the composition applied is comprised between 0.1 and 500 L/Ha. In more preferred embodiments, the amount of the composition applied is comprised between 0.1 and 100 L/Ha. In other even more preferred embodiments, the amount applied can be 0.1 L/Ha, 1 L/Ha, 10 L/Ha, 20 L/Ha, 30 L/Ha, 40 L/Ha, 50 L/Ha, 60 L/Ha, 70 L/Ha, 80 L/Ha, 90 L/Ha or 100 L/Ha.

In particular embodiments of the method for reducing abiotic stress and modulating the metabolism, the dilution object of the invention is applied at the foliar level in an amount comprised between 0.001 and 1000 L/Ha of crops by means of spraying or fumigation, preferably being an amount comprised between 0.1 and 1000 L/Ha, or an amount comprised between 0.001 and 1000 L/Ha of crops is applied at the root level through irrigation, preferably is an amount comprised between 0.1 and 1000 L/Ha.

In preferred embodiments, the amount of dilution applied at foliar level is comprised between 0.1 and 500 L/Ha. In more preferred embodiments, the amount of dilution applied at foliar level is comprised between 0.1 and 100 L/Ha. In other even more preferred embodiments, the amount applied can be 0.1 L/Ha, 1 L/Ha, 10 L/Ha, 20 L/Ha, 30 L/Ha, 40 L/Ha, 50 L/Ha, 60 L/Ha, 70 L/Ha, 80 L/Ha, 90 L/Ha or 100 L/Ha.

In preferred embodiments, the amount of dilution applied at root level is comprised between 0.1 and 500 L/Ha. In other preferred embodiments, the amount of dilution applied at the root level is comprised between 0.1 and 100 L/Ha. In other even more preferred embodiments, the amount applied can be 0.1 L/Ha, 1 L/Ha, 10 L/Ha, 20 L/Ha, 30 L/Ha, 40 L/Ha, 50 L/Ha, 60 L/Ha, 70 L/Ha, 80 L/Ha, 90 L/Ha or 100 L/Ha.

In particular embodiments of the method for reducing abiotic stress and modulating the metabolism, the composition object of the invention or the dilution object of the invention is applied before or after the appearance of an abiotic stress.

Another method for reducing abiotic stress at foliar level to which the invention relates is by placing controlled release diffusers in the proximity of the plant. This controlled release allows the evaporation of the active component of the composition, cis-3-hexenyl propionate, which is more volatile, allowing to obtain an atmosphere rich in this component in the area surrounding the plant. The composition object of the invention applied by this method does not require the use of emulsifying or dispersant additives. The diffusion rate of the volatile active component is regulated by the ratio between cis-3-hexenyl propionate and glycol.

In particular embodiments of the present invention, the composition or dilution object of the invention is applied at least once, and can be applied as many times as necessary to reduce abiotic stress throughout the lifespan of the crop.

A final aspect of the present invention is the use of the composition described herein or the dilution thereof to reduce abiotic stress in plants.

The composition object of the invention is especially advantageous, given that it has the following advantages:
the composition has a low viscosity which allows for easy handling of the product;
the product has just one phase, without the presence of suspended solids, which prevents phase separation or sedimentation in the container;
it has higher efficiency values than those of products available in the state of the art.

### BRIEF DESCRIPTION OF THE FIGURES

To complement the description that is being made and for the purpose of helping to better understand the features of the invention, the following figures have been attached as an integral part of said description, in an illustrative and non-limiting manner :

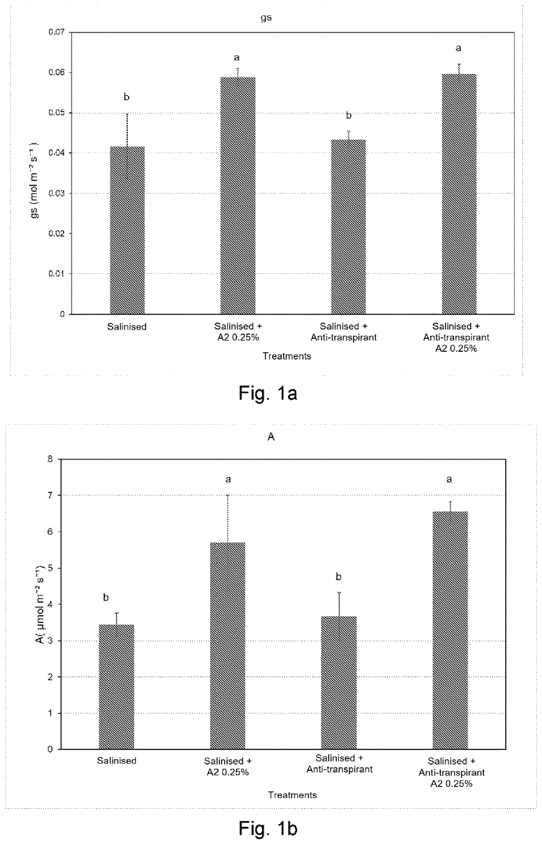

Figure 1a, Stomatal conductance (gs, left) and Figure 1b, photosynthesis (A, right) in citrus trees under conditions of high soil salinity. The first value corresponds to samples without any additional treatment (Salinised) and the following values to samples subjected to several foliar treatments, including a treatment with the composition object of the invention at a concentration of 0.25%, a treatment with an anti-transpirant used in the state of the art and the last value corresponds to the combination of the two foliar treatments. The letters denote statistically significant post hoc differences.

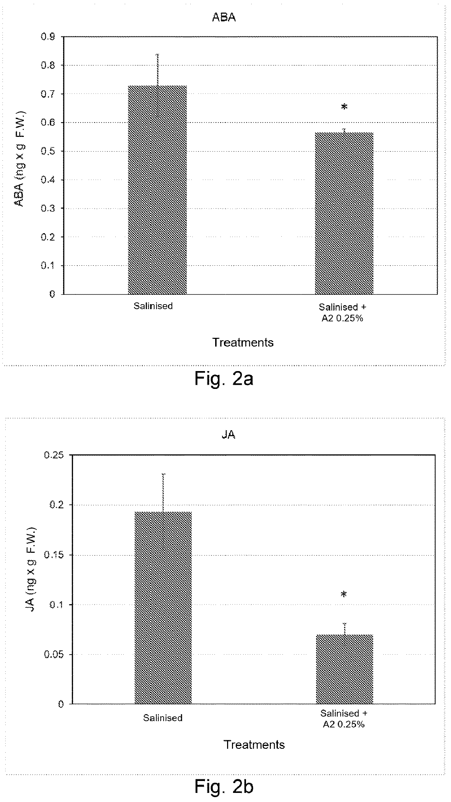

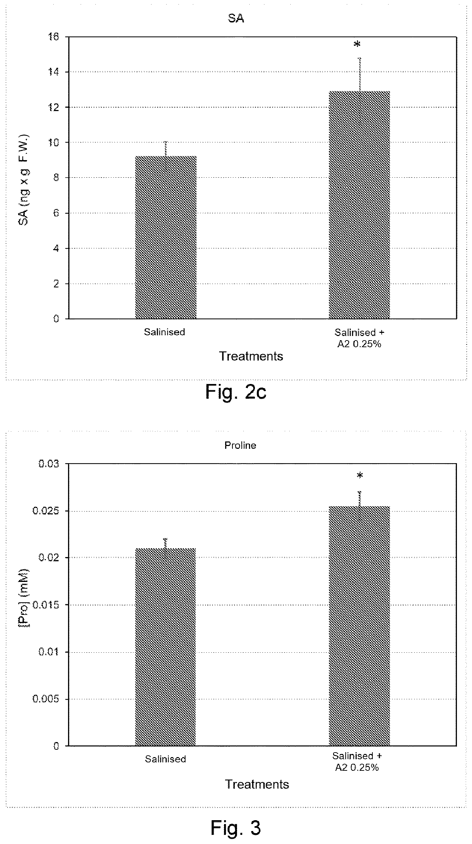

Figure 2a: Endogenous hormonal levels of abscisic acid (ABA), Figure 2b: of jasmonic acid (JA) and Figure 2c: of salicylic acid (SA) in citrus trees under conditions of high soil salinity. The first value corresponds to samples without any additional treatment (Salinised) and the second value to samples subjected to a foliar treatment, with the composition object of the invention at a concentration of 0.25%. Asterisks denote statistical significance (T-test).
Figure 3. Proline levels in citrus trees under conditions of high soil salinity. The first value corresponds to samples without any additional treatment (Salinised) and the second value to samples subjected to a foliar treatment, with the composition object of the invention at a concentration of 0.25%. Asterisks denote statistical significance (T-test).

### PREFERRED EMBODIMENT OF THE INVENTION

A series of tests have been carried out to determine the efficiency of the composition object of the invention.

In particular, in the first stage of the process carried out, two foliar treatments were tested. One treatment was done with a product available on the market and another treatment was done with the composition object of the invention. In particular, the following treatments were tested:
1.- the composition object of the invention, designated for this study as composition A2, diluted in water at a concentration of 0.25% by weight with respect to the total dilution;
2.- an anti-transpirant product that causes greater abiotic stress conditions because it reduces water transpiration through the leaves. Said composition has a concentration of 1% by weight with respect to the total dilution.

These treatments were applied to citrus trees, in particular, to hybrid mandarin (nadorcott) in a commercial plot under high concentrations of salinity in the soil (irrigation water with a concentration of sodium chloride of 90 mmolar) and under high temperature conditions, typical of the Mediterranean summer, between 17 and 45 °C, which represents stress for the crop and a detrimental factor for vegetative development and crop production.

The gas exchange analyses carried out in situ during the month of August showed how the plants treated with the composition object of the invention showed significantly greater stomatal conductance and photosynthesis than the trees that had not received any foliar treatment.

Figures 1a and 1b show how the treatment with the anti-transpirant product used to achieve greater abiotic stress had values equal to those of untreated plants, and furthermore, through the measured parameters, it was detected that the values related to the stress of the plants were less favourable than the untreated salinised plants; however, the application of the composition object of the invention, both alone and in the presence of the anti-transpirant, is capable of increasing the photosynthetic capacity of plants in the previously described unfavourable circumstances.

In this sense, it is observed that, of the two treatments tested in this experiment, the composition object of the invention had a significant capability of regulating the metabolism of plants and alleviating the abiotic stress of plants, maintaining high levels of gs and A. Thus, the composition object of the invention has a much higher efficiency than the anti-transpirant product of the state of the art.

At a biochemical level, it can be seen in Figure 2a how salinised plants treated with composition A2 at a concentration of 0.25% have significantly lower levels of ABA compared to untreated salinised plants.

Likewise, Figures 2b and 2c show how this treatment decreases the levels of jasmonic acid (JA, another hormone related to water stress) and, at the same time, is capable of increasing the concentration of salicylic acid (SA, hormone associated with the production of secondary defense metabolites) with respect to untreated salinised plants.

In this sense, the proline content was analysed, the results of which are shown in Figure 3. It should be noted that the values shown for gas exchange along with the low levels of ABA and the increase in proline are evidence of a differential adaptation of the trees treated with A2, composition object of the invention, and show how these plants have a greater tolerance to stress, the treatment with A2 being the only differential element in this experiment.

The composition object of the invention modifies hormonal levels, adapting the plant response to stress and improving the plant's tolerance to this adverse condition as demonstrated in the figures of gas exchange and proline concentration.

## Claims

1. A composition for reducing abiotic stress in plants, **characterised in that** it comprises in percentage by weight with respect to the total composition:
cis-3-hexenyl propionate between 3% and 50%,
at least one glycol comprised between 49.5% and 85%,
at least one emulsifying additive comprised between 0% and 10%,
at least one dispersant additive comprised between 0% and 2%,
at least one preservative additive comprised between 0% and 2%,
the sum of the components being less than or equal to 100%.

2. The composition according to claim 1, **characterised in that** it has a viscosity between 1 to 100 mPas at a temperature of 25°C.

3. The composition according to claim 1 or 2, wherein the glycol is selected from the group consisting of monoethylene glycol, diethylene glycol, triethylene glycol, monopropylene glycol, dipropylene glycol, glycerin and a combination thereof

4. The composition according to any one of claims 1 to 3, wherein the emulsifying additive is selected from the group consisting of lecithin, polysorbate, ammonium phosphate, guar flour, glyceryl esters, fatty acid salts, fatty acid monoglycerides or diglycerides, citric acid, tartaric or acetic esters, polyglyceride esters, fatty acids, glutamates and a combination thereof.

5. The composition according to any one of claims 1 to 4, wherein the dispersant additive is inorganic and is selected from the group consisting of sorbic acid, sodium sorbate, benzoic acid, sodium benzoate, formic acid, sodium formate, boric acid, potassium tetraborate, acetic acid, sodium or potassium acetate, lactic acid, chitosan and a combination thereof.

6. The composition according to any one of claims 1 to 5, wherein the dispersant additive is organic and is selected from the group consisting of polyvinyl alcohol, methyl cellulose, methyl hydroxypropyl cellulose, ethyl cellulose, sodium carboxymethyl cellulose and a combination thereof.

7. The composition according to any one of claims 1 to 6, wherein the preservative additive is selected from the group consisting of sorbic acid, sodium sorbate, benzoic acid, sodium benzoate, formic acid, sodium formate, boric acid, potassium tetraborate, acetic acid, sodium or potassium acetate, lactic acid, chitosan and a combination thereof.

8. A method for producing a composition for reducing abiotic stress in plants defined in any one of claims 1 to 7, **characterised in that** it comprises mixing an amount comprised between 3% and 50% of cis-3-hexenyl propionate with an amount comprised between 49.5% and 85% of at least one water-soluble glycol while stirring at a speed of 100 to 300 rpm at a temperature between 20 to 30°C for a time of 20 to 30 minutes, the amounts being by weight with respect to the total of the composition.

9. The production method according to claim 8, **characterised in that** it comprises an additional previous step wherein:
at least one emulsifying additive in an amount comprised between 0.1% and 10%, and/or
at least one dispersant additive in an amount comprised between 0.05% and 2%, and/or
at least one preservative additive in a concentration between 0.05% and 2%, the amounts being by weight with respect to the total composition;
are added to an amount comprised between 49.5% and 85% of at least one water-soluble glycol and are mixed at a speed of 100 to 300 rpm for a time between 20 and 60 minutes at a temperature between 20 and 30°C.

10. A dilution, **characterised in that** it comprises an amount between 0.01% and 10% of a composition defined in any one of claims 1 to 7 in a liquid medium, the amount being by weight with respect to the total dilution.

11. The dilution according to claim 10, wherein the liquid medium is pure water.

12. A method for reducing abiotic stress in plants, **characterised in that** it comprises applying at least once the composition defined in any one of claims 1 to 7 or a dilution defined in any one of claims 10 to 11 to agricultural crops by means of spraying, fumigation, controlled evaporation or irrigation.

13. The method according to claim 12, wherein the composition defined in any one of claims 1 to 7 or the dilution defined in any one of claims 10 to 11 is applied before or after the appearance of an abiotic stress.

14. The method according to claim 12 or 13, wherein the composition defined in any one of claims 1 to 7 is applied at foliar level in an amount comprised between 0.001 and 1000 L/Ha of crops by controlled evaporation.

15. The method according to claim 12 or 13, wherein the dilution defined in any one of claims 10 to 11 is applied at foliar level in an amount comprised between 0.001 and 1000 L/Ha of crops by means of pulverisation or fumigation, or an amount comprised between 0.001 and 1000 L/Ha of crops is applied at the root level by means of irrigation.

16. A use of a composition defined in any one of claims 1 to 7 for reducing abiotic stress in plants.

17. The use of a dilution defined in any one of claims 10 to 11 for reducing abiotic stress in plants.
